# EUROPEAN PATENT SPECIFICATION

(11) **EP 2 451 518 B1**
(45) Date of publication and mention of the grant of the patent: **11.10.2017**
(21) Application number: 10796573.3
(22) Date of filing: 02.07.2010
(51) Int. Cl.: A61M 16/06, A62B 18/02

(54) **MULTIPLE CHAMBERS MASK**
MEHRFACHE KAMMERMASKE
MASQUE À CHAMBRES MULTIPLES

(30) Priority: 06.07.2009 AU 2009903135
(43) Date of publication of application: 16.05.2012
(73) Proprietor: Compumedics Medical Innovation Pty Ltd, Abbotsford, Victoria 3067 (AU)
(72) Inventor: ZIV, Hedi, Burwood, Victoria 3125 (AU); MCCOOEY, Conor, Brighton, Victoria 3186 (AU); SLABBERT, Rikus, Hawthorn East, Victoria 3123 (AU)
(74) Representative: Handsome I.P. Ltd
(86) International application number: PCT/AU2010/000842
(87) International publication number: WO 2011/003130

(56) References cited:
- GB-A- 1 379 429
- JP-A- S58 136 345
- US-A- 5 657 752
- US-A1- 2006 260 614
- US-A1- 2007 006 879
- US-A1- 2007 089 749

## Description

### Field of the Invention

The present invention relates to masks for the provision of gases to subjects, in particular, masks for delivery of pressurised gas to the airways of a subject.

### Background

There are a number of treatments that require the use of a mask for the delivery of gas or air to a subject. For example, in the treatment of sleep apnoea, gas is often delivered at continuous positive airway pressure (CPAP) wherein gas is supplied continuously at a pressure greater than ambient, or variable positive airway pressure (VPAP) wherein gas is supplied at varying pressures, or other such methods such as BiPAP wherein gas is supplied at two pressures, or APAP wherein gas is supplied at pressures determined automatically by the delivery system to a sleeping subject through a mask to keep the patient's airways open for effective respiration. Often gas must be delivered through a mask for sustained time periods, for example, through a whole overnight period of sleep.

It is important for continuous therapeutic benefit from pressurised gas or air that a mask assembly be comfortable and relatively leak-proof when positioned for gas delivery. The mask must be comfortable so that a subject achieves the therapeutic benefit of relatively unbroken sleep periods. Masks known in the art often incorporate an interface such as a cushion or pillow, made of soft material to be most comfortable for a subject during long periods of use, but are capable of sealing adequately to minimise leaks. A nasal cushion, for example, is sealingly engaged with the bridge and sides of the nose, and the upper lip to provide a leak-proof conduit for pressurised gas delivery.

Prior art mask assemblies have been designed to allow some gas leakage from the mask assembly during gas delivery. An unfortunate consequence is the acoustic noise attributable to these leaks may cause a subject to have difficulty falling asleep and then being aroused from sleep during positive airway pressure treatment Arousals, in turn, may limit the efficacy of the treatment. Further, the acoustic noise also may affect the sleeping of nearby persons. What is needed is an interface for pressurised gas delivery in any of CPAP/VPAP/BiPAP/APAP system wherein the interface delivers gas to the airways of a subject with reduced acoustic noise.

US2006/0260614A1 describes a breathing mask including a first chamber and a passage means for discharging gas to atmosphere via a gap portion.

### Summary of the Invention

The invention is defined by the appended claims.

It is an object of the invention to provide an interface for a gas-delivery mask that generates reduced acoustic noise during gas delivery. The present invention most advantageously provides solutions to the problem of noisy masks when pressurised air or gases is provided to a subject. The mask is both comfortable and less noisy than masks currently found in the art. The invention provides an interface for a mask for delivery of pressurised gas to a subject incorporating means to reduce the acoustic noise generated from the pressurised gas. The interface may be a nasal cushion or other means, such as a face cushion. The noise reduction means comprises of a baffling means. The baffling means preferably comprises of a porous barrier defining volumes in gaseous communication within the nasal interface. Preferably there are two adjacent volumes defined by a porous barrier.

Surprisingly, the acoustic noise of multiple adjacent volumes is reduced compared with a single volume. The invention has many possibilities for uses in different applications whenever pressurised gas is delivered to a subject. Some examples are for delivering oxygen in aircraft, or for delivering gaseous anaesthetics or therapeutic substances suitable for gas delivery. In particular, the invention will be most advantageously used for gas delivery for application of CPAP therapy. The reduced acoustic noise generated by a gas-delivery mask according to the invention enables a more efficient sleep with fewer arousals.

In a first aspect, the invention provides a mask for delivering pressurised gas to a subject, the mask comprising a first aperture in gaseous communication with a pressurised gas supply; a second aperture for efflux of pressurised gas; a porous barrier defining at least two chambers, the first chamber being in communication with the first aperture and the second chamber being in communication with the second aperture, characterised in that the two chambers are in gaseous communication via a plurality of pores arranged in the barrier, the plurality of pores arranged to cooperate, in use, to decrease the acoustic noise attributable to the efflux of the pressurised gas. Preferably the second chamber communicates through apertures for gaseous flow into the ambient air. Preferably, the mask includes a conduit in communication with the gas supply and the mask. Preferably, the apertures define a tapered shape. Most preferably, the mask includes a nasal cushion. Preferably, the nasal cushion comprises of a pliable material. The description also discloses a method of providing pressurised gas to a subject, the method comprising the steps of providing a mask according to the first aspect; supplying gas from a pressurised source to the mask; transmitting the gas into the first chamber of the mask at a first pressure; transmitting the gas into the second chamber of the mask at a second pressure; and transmitting the gas from the second chamber through an exit.

Other features are recited in the appended claims.

### Brief Description of the Figures

Figure 1 shows a longitudinal transverse view through a mask assembly having a nasal cushion interface according to the invention.
Figure 2 shows a transverse view of a few slot leak aperture design of a mask assembly having a nasal cushion according to the invention.
Figure 3 shows a transverse view of multiple leak apertures design of a mask assembly having a nasal cushion according to the invention.
Figure 4 shows a comparison between a parallel wall pore (a) and a tapered pore (b).

### Detailed Description of the Invention and Most Preferred Embodiment

The invention advantageously exploits the acoustic muffling effect of adjacent chambers in gaseous communication, the chambers defined by a baffling means. The baffle means comprises of a barrier incorporating pores of defined cross-sectional areas. Preferably the baffle incorporates a plurality of small pores which cooperate to decrease the acoustic noise attributable to the gas flow. More preferably, the pores are tapered. The pores may be frusto-conical in shape. The baffling means may comprise of multiple small tapered pores. Surprisingly, the acoustic noise of the multiple small tapered pores is reduced compared with fewer, larger pores creating the same total cross-sectional area. The acoustic noise of the tapered pores or frusto-conical pores may be reduced compared with cylindrical pores. The reduced acoustic noise enables a more efficient sleep with fewer arousals.

It is known in the art that for two pores in series design, compared to a single pore design, having the same overall pressure drop at the same flow rate, the two pores should have a diameter of about 2<1/4> d = 1.19 d, where d is the diameter of a single pore. The velocity of gas movement through the pores reduces to 8-^2 V where V is the original mean velocity. Given that the sound power is proportional to the product of pore area and velocity to the 8th power, the acoustic power emission per pore should be reduced by a factor of 8V2 = 11.3 or about 10 dB. In practice, the internal pore noise is not fully absorbed. A further advantage is that the reduction in velocity attributable to chambers in gaseous communication would be expected to reduce the external draught effect at the leak site.

The pressure difference of gas in volumes in gaseous communication is proportional to the cross- sectional area defined by the apertures of the pores. Optimal noise suppression may be effected by reducing the magnitude of pressure drop, by introducing intermediate pressure drops in multiple intermediate chambers, and implementing multiple small tapered pores, although either feature may be incorporated in an embodiment to achieve some noise suppression.
The figures illustrate embodiments of the invention. It will be understood that there are many other possible embodiments of the invention and that the invention is limited only by the scope of the claims appended hereto.

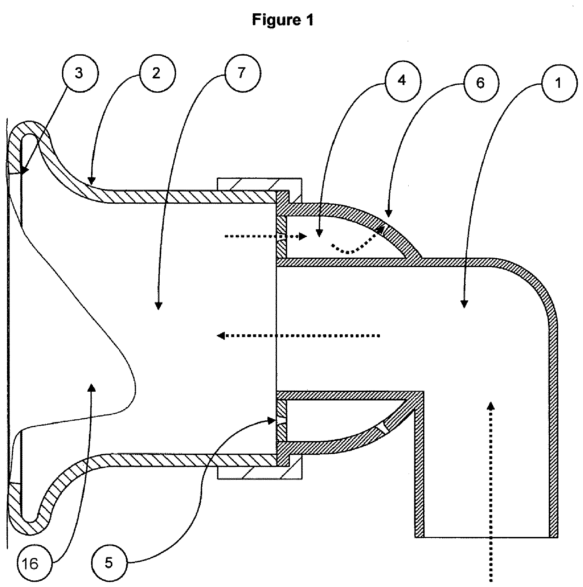

Figure 1 shows an embodiment of the invention as a dual chamber gas-delivery mask. The left side of the drawing shows a nasal mask in operative position on the face of a subject, with the subject's nose (16) extending into a first chamber (7) defined by the nasal interface. In this embodiment, the interface is a nasal cushion (2). Alternative embodiments such as facial cushions or pillows may also be used. The PAP (positive airway gas pressure) is supplied via a hose (1) into the nasal cushion defining a chamber (7), the nasal cushion engaging the subject's face with a flexible sealing membrane (3). The pressure differential between the above-ambient pressure gas from the hose (1) flowing into the nasal chamber (7) and a second chamber (4) in gaseous communication with the first chamber (7) results in gas flux between the chambers.

A further gas pressure differential between the second chamber (4) and the ambient air results in gas flux into the air. The pressurised gas from the second chamber (4) flows to the ambient air via pores (6) which are preferably tapered or frusto-conical in shape, but may also take other shapes as necessary, such as being cylindrical. The gas may flow through pores (5) in a porous baffle defining the boundary between the chambers (7), (4). Figure 1 shows two chambers in gaseous communication but further chambers may be incorporated into the mask. The invention includes more than two chambers, if necessary, according to the application. The pathway of the PAP through the supply hose and interface is shown by the dotted lines in Figure 1. The total area of pores (5) between the main chamber (7) and the secondary chamber (4); and the total area of pores (6) between the secondary chamber (4) and the ambient air determines the pressure difference between the main chamber (7) and the secondary chamber (4), as well as the pressure difference between the secondary chamber (4) and the ambient air. The reduction of pressure difference between the two chambers or the secondary chamber and the ambient air, generates a lower flow through the pores and thus creates less turbulence, resulting in a reduced acoustic noise level.

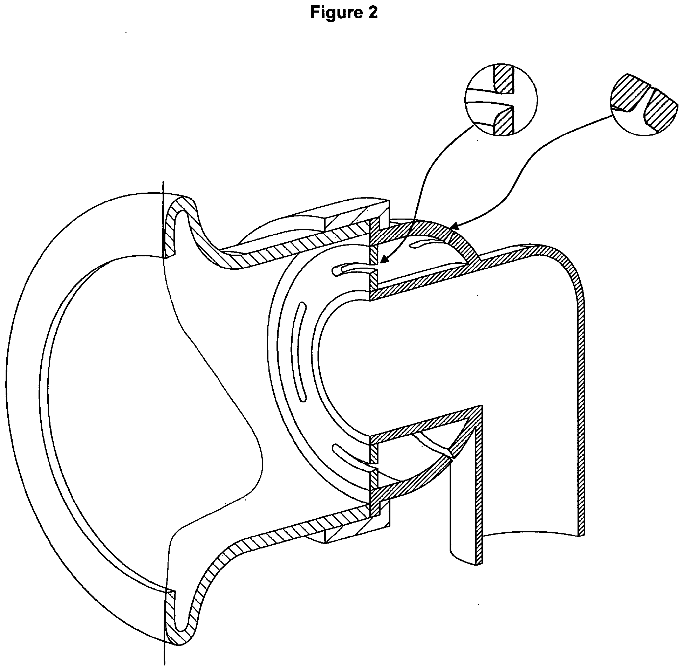

Figure 2 shows an embodiment of the invention having multiple large pores for the gas leak.

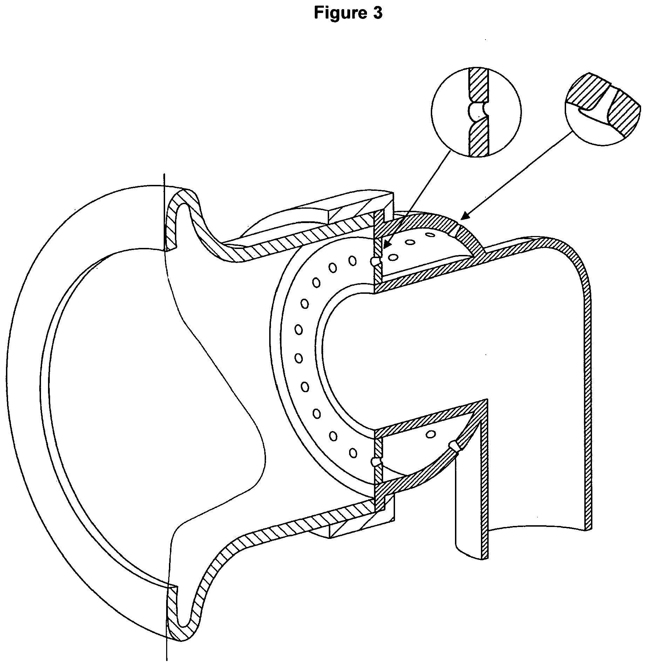

Figure 3 displays small multiple tapered pores for the gas leak.

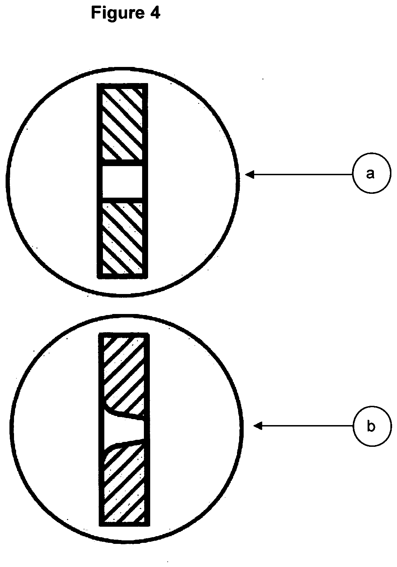

Figure 4a shows an embodiment of the invention having a cylindrical pore.

Figure 4b a further embodiment having a tapered pore. The smooth entrance to the aperture reduces turbulence of the gas being released; the tapered shape decelerates the gas flow, resulting in a reduced acoustic noise level.

## Claims

1. A mask for delivering pressurised gas to a subject, the mask comprising a first aperture in gaseous communication with a pressurised gas supply; a second aperture (6) for efflux of pressurised gas; a porous barrier defining at least two chambers, the first chamber (7) being in communication with the first aperture and the second chamber (4) being in communication with the second aperture, **characterised in that** the two chambers are in gaseous communication via a plurality of pores (5) arranged in the barrier, the plurality of pores arranged to cooperate, in use, to decrease the acoustic noise attributable to the efflux of the pressurised gas.

2. The mask of claim 1 further comprising a conduit (1) in communication with a gas supply.

3. The mask of either one of claims 1 and 2, further comprising a nasal cushion (3).

4. The mask of any preceding claim, wherein the wherein the second aperture includes a plurality of pores (5) being frusto-conical in shape.

5. The mask of any preceding claim, wherein the second chamber (4) is in gaseous communication with the ambient air.

6. The mask of any preceding claim, wherein the first chamber (7) is arranged for engagement with at least a portion of the face of a subject.

7. The mask of any preceding claim, wherein the first chamber (7) comprises of a pliable material.

8. The mask according to any preceding claim being an anaesthetic gas delivery mask.

## Patentansprüche

1. Maske für die Abgabe von unter Druck stehendem Gas an ein Subjekt, wobei die Maske eine erste Öffnung umfasst in Gasverbindung mit einer Quelle für unter Druck stehendes Gas, eine zweite Öffnung (6) für das Ausströmen von unter Druck stehendem Gas, eine poröse Barriere, welche wenigstens zwei Kammern definiert, wobei die erste Kammer (7) in Verbindung steht mit der ersten Öffnung und die zweite Kammer (4) in Verbindung steht mit der zweiten Öffnung, **dadurch gekennzeichnet, dass** die beiden Kammern in Gasverbindung stehen über eine Mehrzahl von Poren (5), angeordnet in der Barriere, wobei die Mehrzahl von Poren eingerichtet ist, dazu beizutragen, dass im Gebrauch das akustische Geräusch, welches durch das Ausströmen des unter Druck stehenden Gases verursacht wird, verringert wird.

2. Maske nach Anspruch 1 weiterhin umfassend eine Leitung (1) in Verbindung stehend mit einer Gasquelle.

3. Maske nach einem der Ansprüche 1 oder 2, weiterhin umfassend ein Nasalkissen (3).

4. Maske nach einem der vorhergehenden Ansprüche, bei der die zweite Öffnung eine Mehrzahl von Poren (5) umfasst, welche eine kegelstumpfförmige Form aufweisen.

5. Maske nach einem der vorhergehenden Ansprüche, bei der die zweite Kammer (4) in Gasverbindung steht mit der Umgebungsluft.

6. Maske nach einem der vorhergehenden Ansprüche, bei der die erste Kammer (7) eingerichtet ist für einen Eingriff mit wenigstens einem Abschnitt der Fläche eines Subjekts.

7. Maske nach einem der vorhergehenden Ansprüche, bei der die erste Kammer (7) ein biegsames Material umfasst.

8. Maske nach einem der vorhergehenden Ansprüche, welche eine Maske für die Abgabe von Betäubungsgas ist.

## Revendications

1. Masque pour distribuer à un sujet un gaz mis sous pression, le masque comprenant une première ouverture en communication gazeuse avec une alimentation en gaz mis sous pression ; une seconde ouverture (6) pour la sortie de gaz mis sous pression ; une barrière poreuse définissant au moins deux chambres, la première chambre (7) étant en communication avec la première ouverture et la seconde chambre (4) étant en communication avec la seconde ouverture, **caractérisé par le fait que** les deux chambres sont en communication gazeuse par l'intermédiaire d'une pluralité de pores (5) agencés dans la barrière, la pluralité de pores étant agencée pour coopérer, en utilisation, pour diminuer le bruit acoustique pouvant être attribué à la sortie du gaz mis sous pression.

2. Masque selon la revendication 1, comprenant en outre un conduit (1) en communication avec une alimentation en gaz.

3. Masque selon l'une des revendications 1 et 2, comprenant en outre un coussinet nasal (3).

4. Masque selon l'une quelconque des revendications précédentes, dans lequel la seconde ouverture comprend une pluralité de pores (5) de forme tronconique.

5. Masque selon l'une quelconque des revendications précédentes, dans lequel la seconde chambre (4) est en communication gazeuse avec l'air ambiant.

6. Masque selon l'une quelconque des revendications précédentes, dans lequel la première chambre (7) est agencée pour être en engagement avec au moins une partie du visage d'un sujet.

7. Masque selon l'une quelconque des revendications précédentes, dans lequel la première chambre (7) est constituée d'une matière pliable.

8. Masque selon l'une quelconque des revendications précédentes, qui est un masque de distribution de gaz anesthésique.
